# EUROPÄISCHE PATENTSCHRIFT

(11) **EP 1 105 079 B1**
(45) Veröffentlichungstag und Bekanntmachung des Hinweises auf die Patenterteilung: **16.04.2003**
(21) Anmeldenummer: 99936223.9
(22) Anmeldetag: 13.08.1999
(51) Int. Cl.: A61F 13/15

(54) **HYGIENE-EINLAGE FÜR MÄNNER**
HYGIENIC PAD FOR MEN
SERVIETTE HYGI NIQUE DESTIN E AUX HOMMES

(30) Priorität: 19.08.1998 CH 170398
(43) Veröffentlichungstag der Anmeldung: 13.06.2001
(73) Patentinhaber: Zanetti, Johann, 3000 Bern 9 (CH)
(72) Erfinder: Lehmann-Ott, André, 6344 Meierskappel (CH); Lehmann-Ott, Monique, 6344 Meierskappel (CH)
(74) Vertreter: Eder, Carl E.
(86) Internationale Anmeldenummer: CH9900375
(87) Internationale Veröffentlichungsnummer: WO00010495

(56) Entgegenhaltungen:
- EP-A- 0 605 017
- EP-A- 0 705 586
- DE-A- 19 542 586
- US-A- 3 570 491
- US-A- 4 605 404
- US-A- 5 514 104

## Beschreibung

Die Erfindung betrifft eine Hygiene- Einlage in Männer-Unterhosen zur Verbesserung der Hygiene, besonders bei Tropfinkontinenz, nach dem Oberbegriff des Patentanspruchs 1.

Tropfinkontinenz ist ein sowohl bei Frauen als auch bei Männern weit verbreitetes Problem. Die Bewegungsfreiheit kann durch dieses Leiden selbst bei nur leichter Erkrankung erheblich beeinträchtigt werden, indem die Leute wegen den Gerüchen, die dadurch verbreitet werden, die Nase rümpfen und einen Bogen um die entsprechende Person machen. Gerade aus diesem Grund ist es für die Betroffenen sehr wichtig, adäquate Hilfsmittel zur Verfügung zu haben. Es gibt unterschiedliche Grade zweier verschiedener Typen von Inkontinenz, der Stress- und der Dranginkontinenz. Besonders verbreitet ist die leichte Stressinkontinenz, bei der es schon bei ruckartigen Bewegungen, zum Beispiel beim Niesen, beim Lachen, beim Treppensteigen oder beim Anheben von schwereren Gegenständen, zu leichtem Urinverlust kommen kann. Ebenfalls bekannt ist ein abgeschwächter Harnstrahl und das mit diesem oft verbundene, für den Betroffenen äusserst mühsame sogenannte Nachtröpfeln.

Es gibt bereits einige Hilfsmittel für Menschen, die an einer solchen leichten Inkontinenz, insbesondere in Form des Nachtröpfelns, leiden. Das in diesem Fall anzuwendende Hilfsmittel hat die Aufgabe, die Tröpfchen, die nach dem Urinieren aus der Harnröhre ausfliessen, aufzufangen, ohne dass der Betroffene ein Nässegefühl empfindet und ohne dass die Unterhose oder gar die Hose in Mitleidenschaft gezogen werden, so dass nicht nur die Unterhosen, sondern auch die anderen Hosen täglich gewechselt werden müssen, damit man die Umwelt nicht mit unangenehmen Gerüchen belästigt.

Als Lösung gibt es zum Beispiel für mittel bis schwer inkontinente Männer diverse mehrfach einsetzbare Windeln. Diese Windeln bestehen auf ihrer Aussenseite meistens aus Gummi oder Kunstgummi und innen aus einer dicken Schicht Kunstfasern. Der Nachteil dieser Windeln ist, dass sie sehr stark auftragen. Zudem ist mit dem Waschen der Windeln ein sehr grosser Aufwand verbunden. Weiter sind mehrfach einsetzbare Windeln bekannt, welche mit einer speziellen Netzhose oder einer stabilisierenden Unterhose getragen werden müssen.

Alle diese Hilfsmittel für inkontinente Männer sind jedoch umständlich in der Handhabung, tragen teilweise stark unter der Kleidung auf, verursachen bei Bewegungen störende Geräusche, bringen den Träger zum Schwitzen und tragen den anatomischen Verhältnissen im Genitalbereich von Männern zu wenig Rechnung. Sie sind keine brauchbaren Hilfsmittel für Männer, welche nur leicht inkontinent sind und somit nur ab und zu ein paar Tröpfchen Urin verlieren.

Als weitere Lösung wurden auch bereits Binden oder Einlagen, die vorne im Schritt der Unterhose befestigt werden, vorgeschlagen. Der Vorteil solcher Einlagen ist, dass diese bei richtiger Formgebung und Dimensionierung nicht auftragen und somit nicht zu sehen sind. Ein weiterer Vorteil dieser Einlagen ist, dass sie zu normaler Unterwäsche getragen werden können. Die bekannten Damenbinden, welche für die Aufnahme der im Zusammenhang mit der Menstruation auftretenden physiologischen Flüssigkeiten vorgesehen sind, können jedoch von Männern nicht verwendet werden, da die männlichen Genitalien im Schritt bedeutend mehr Platz benötigen als die weiblichen.

Eine solche Einlage für Unterhosen wird zum Beispiel in der Schweizer Patentschrift CH 681 688 vorgeschlagen. Die dort beschriebene Einlage besteht aus einer inneren Schicht eines saugfähigen Materials, welche mit einer äusseren Schicht aus einem rückstellelastischen Material fest verbunden ist. Diese elastische Schicht ist mit einem Dauerkleber versehen, mit dessen Hilfe die Einlage in der Unterhose befestigt werden kann. Die Einlage ist jedoch relativ schmal und bleibt in ihrer Form in der Unterhose relativ eben, so dass sie für leicht bis mittelschwer inkontinente Männer nicht brauchbar ist.

Weiter schlägt die deutsche Offenlegungsschrift DE 196 45 755 eine Einlage vor, welche ebenfalls im Schritt der Unterhose getragen wird und aus einern länglichen Hauptteil und zwei kleineren, flexiblen Klappen auf der Seite besteht. Die Einlage wird mit je einem Klebstreifen auf der Rückseite und auf den beiden seitlichen Klappen, welche um die Ränder der Unterhose gebogen werden, in der Unterhose befestigt. Auch diese Einlage ist für Männer nicht geeignet, da die Form nicht speziell für sie ausgestaltet ist. Zudem kann diese Einlage nicht in allen Typen von Unterhosen verwendet werden, insbesondere nicht in Boxershorts.

Die Schweizer Patentschrift CH 680 647 schliesslich schlägt eine auswechselbare Einweg-Einlage für Unterhosen vor, welche in eine mehrfach verwendbare, waschbare und eigens für diesen Zweck geformte Unterhose eingeführt wird. Diese Einlage hat den grossen Nachteil, dass sie nicht zu normaler Unterwäsche getragen werden kann. Zudem ist auch die Formgebung dieser Einweg-Einlage nicht speziell an die anatomischen Verhältnisse der Männer angepasst.

Aus der EP-A2-0 605 017 ist allgemein eine Hygiene-Einlage bekannt, deren Mittelteil etwa rechteckig ausgebildet ist und deren sich an die Längsenden des Mittelteils anschliessende Endbereiche etwa halbkreisförmig ausgebildet sind.

Eine Hygiene-Einlage für Männer gemäss dem Oberbegriff des Patentanspruchs 1 ist aus der DE-A1-195 42 586 bekannt. Diese Hygiene-Einlage besteht aus saugfähigem Material, wobei die aus formbarem Material ausgebildete Hygiene-Einlage in ihrer Grösse im wesentlichen der Glans entspricht und während des Gebrauchs zwischen Glans und Vorhaut angeordnet ist. Diese Hygiene-Einlage hat an einer Ober- und Unterseite einen weichen Vliesstoff, wobei zum Inneren der Hygiene-Einlage jeweils eine Lage stark saugfähiger Baumwoll- oder Papierwatte mit Viskosefastern folgt. In der Mitte der Hygiene-Einlage befindet sich eine Lage aus Kohlepartikeln. Die bekannte Hygiene-Einlage ist, wie zuvor erwähnt, zwischen Vorhaut und Glans angeordnet und damit umständlich einzusetzen und vor jedem Urinieren zu entfernen.

Die vorliegende Erfindung stellt sich nun die Aufgabe, eine einfach anwendbare. zuverlässig den tröpfchenweise austretenden Urin aufsaugende, im Schrittbereich der Unterhose getragene Einlage, insbesondere für leicht inkontinente Männer, zu schaffen, welche die Nachteile der bisherigen Einlagen und der anderen Hilfsmittel für diese Kategorie von inkontinenten Männern nicht aufweist. Dabei wird durch eine leichte Inkontinenz unkontrolliert Urin abgegeben. Für die Einlage soll eine Form gewählt werden, die in allen verbreiteten Typen von Unterhosen auf einfache und sichere Weise eingesetzt werden kann, bequem zu tragen ist und zu einem günstigen Preis auf den Markt gebracht werden kann.

Die Aufgabe wird mit Hilfe der erfindungsgemässen Merkmale des Patentanspruchs 1 gelöst. Vorteilhafte Weiterbildungen der Erfindung sind Gegenstand der jeweils abhängigen Ansprüche.

Die vorgeschlagene Einlage für Unterhosen ist somit besonders für Männer gedacht, welche an leichter Inkontinenz, der sogenannten Tröpfcheninkontinenz, leiden und zugleich auf Hygiene grossen Wert legen. Die vorgeschlagene Einlage besteht aus einer mittels eines hydrophilen Vlieses abgedeckten, saugfähigen Mittelschicht, die auf eine äusseren Schicht in form einer wasserdichten schicht oder eines hydrophoben Vlieses aufliegt. Auf der Aussenseite der wasserdichten Schicht Folie oder des hydrophoben Vlieses ist die durchgehende Klebefläche (streifenartig und dadurch luftdurchlässig) aufgetragen, womit die Einlage in der Unterhose befestigt werden kann. Die Form der vorgeschlagenen Einlage ist so ausgestaltet, dass die Einlage unter dem Einfluss der Körperwärme und der Form der Unterhose in eine Schalenform übergeht. Diese Form ermöglicht es, dass der Penis stets in der Mitte der Unterhose und somit in der Einlage gehalten wird. Einschnitte im

Rand der Einlage sorgen dafür, dass die Schalenform ohne Verwerfungen gebildet werden kann. Die Einlage ist zudem so geformt, dass sie in sämtlichen Unterhosen getragen werden kann. Weiterhin ist in einer bevorzugten Ausführungsform vorgesehen, die Einlagen so zu gestalten, dass ein stehendes Urinieren, zum Beispiel in öffentlichen Pissoirs, problemlos möglich wird. Dies heisst, die Einlagen sollen so geformt sein, dass der Penis ohne weiteres durch den Hosenschlitz hinausgeführt werden kann, ohne dass man die Hosen ganz hinunterlassen und sich auf die Toilette setzen muss.

Die Erfindung wird im folgenden anhand eines Ausführungsbeispiels näher erläutert. Sie ist unter anderem auch in einer Zeichnung beschrieben. So zeigt:
Fig. 1 die Innenseite der erfindungsgemässen Einlage in Aufsicht; und
Fig. 2 einen der besseren Übersicht halber auseinandergezogenen Schnitt entlang der Linie A- A in Fig. 1.

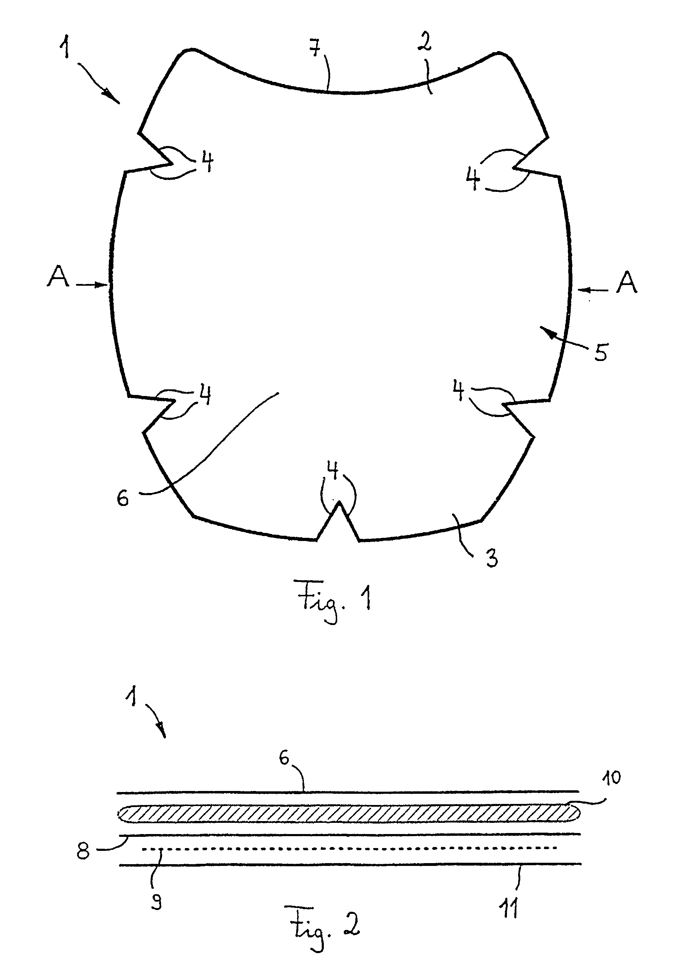

Figur 1 zeigt somit ein Ausführungsbeispiel der erfindungsgemässen Unterhosen-Einlagen. Diese Einlage 1, welche die Form einer unregelmässigen Ellipse hat, weist auf den langen Seiten der Ellipse je zwei und auf der unteren kurzen Seite 3 der Ellipse einen v-förmigen Einschnitt 4 auf. Diese v-förmigen Einschnitte 4 haben die Aufgabe, das Aufstellen der Ränder 5 der Einlage 1 verwerfungsfrei zu ermöglichen und somit die Bildung einer schalenförmigen Vertiefung unter dem Einfluss der Körperwärme in der Unterhose zuzulassen. Diese schalenförmige Vertiefung hält das männliche Glied in der Mitte und sorgt somit dafür, dass die aus der Blase austretenden Urintröpfchen von der Einlage 1 vollständig aufgefangen werden können und somit keine Feuchtigkeit die Unterhose oder gar die Hose in Mitleidenschaft zieht.

Die erfindungsgemässe Einlage 1 besteht aus mindestens zwei unterschiedlichen Lagen, nämlich aus einer äusseren Schicht 8 in form eines wasserdichten aber dennoch luftdurchlassigen Schicht oder eines hydrophoben Vlieses , auf die eine einen Klebstoff aufweisende Klebefläche 9 aufgebracht ist, und einer mittleren, saugfähigen Schicht 10, welche auch Mittelschicht genannt wird. Gemäss dem in Figur 2 gezeigten Ausführungsbeispiel weist die Einlage 1 zusätzlich zu der Schicht 8 und der saugfähigen Schicht 10 eine innerste Schicht in Form eines die Innenseite der Schicht 10 abdeckenden Vlieses 6 auf. Somit sind auf der Innenseite der ausseren Schicht 8 die saugfähige, mittlere Schicht 10 und das Vlies 6 angebracht. Die Schicht 10 ist luftdurchlässig und mittels superabsorbierenden Fasern derart ausgebildet, dass sie darauf auftreffende Flüssigkeit nach innen leitet. Das Vlies 6 ist hydrophil und die aussere Schicht 8 hydrophob ausgebildet. Im ungebrauchten Zustand ist die Klebefläche 9 auf der äusseren Schicht 8 mit Vorteil mittels eines Schutzstreifens 11 bedeckt, welcher vor Gebrauch durch einfaches Wegziehen problemlos entfernt werden kann. Dieser Schutzstreifen 11 besteht vorzugsweise aus Silikonpapier. Diese Technik ist bereits bekannt und wird zum Beispiel bei Damenbinden angewendet.

Die erfindungsgemässe Einlage 1 ist auf der oberen kurzen Seite 2 halbmondförmig ausgespart. Dieser halbmondförmige Ausschnitt 7, der in der Unterhose nach oben weist, ermöglicht es dem Benutzer, den Penis beim stehenden Urinieren, zum Beispiel in einer öffentlichen Toilette, auf einem Pissoir oder im Freien, ohne Probleme durch den Deckverschluss der Unterhose und den Reissverschluss der Hose zu schieben. Dies ist für inkontinente Männer besonders wichtig, so können sie ohne Einschränkung die normalen sanitären Einrichtungen benutzen.

Die Dicke der saugfähigen Mittelschicht 10 liegt zwischen 2 und 4 mm, vorzugsweise aber zwischen 2,5 und 3 mm. Die Breite der Einlage 1 beträgt 100 bis 120 mm, vorzugsweise aber 110 mm und die Länge der Einlage 1 beträgt 110 bis 130 mm, vorzugsweise aber 120 mm.

Die vorgeschlagene Unterhosen-Einlage 1 weist gegenüber den bekannten Hilfsmitteln wesentliche Vorteile auf: So kann zum Beispiel die Einlage durch ihre Universalform in sämtlichen Formen von Unterhosen getragen werden. Zusätzlich ist auch die Anwendung der Einlage sehr einfach, indem sie im Schritt der Unterhose in der unteren Hälfte nach dem Ablösen des Schutzstreifens 11 auf einfache Weise eingeklebt werden kann. Auf diese Art und Weise häit die Einlage für einen ganzen Tag. Zudem sorgt die spezielle Form der Einlage wie bereits erwähnt dafür, dass sie unter dem Einfluss der Unterhose und der Körperwärme eine Schalenform einnimmt und der Penis somit optimal in der Mitte gehalten wird und kein austretender Urin die Unterhose oder gar die Hose beeinträchtigt. Die saugfähige Mittelschicht 10 der Einlage ist sehr weich und saugfähig. Sie vermittelt dadurch dem Benutzer den ganzen Tag ein angenehmes Gefühl von Wohlbehagen sowie von optimaler Hygiene, weil die Feuchtigkeit nach innen geleitet wird und nicht mehr auf der Haut spürbar ist. Durch das nach innen Leiten der Flüssigkeit erreicht man nämlich, dass das auf die saugfähige Mittelschicht 10 gelegte Vlies 6 trocken bleibt. Zudem schwitzt der Benutzer infolge der relativ dünnen Einlage nicht. Schliesslich wird dank der besonderen Ausgestaltung der erfindungsgemässen Einlage 1 ein Urinieren auch auf Pissoiren in öffentlichen Anlagen möglich. Diese Vorteilskombination weist keine der bereits bekannten Hilfsmittel für leicht inkontinente Männer, sogenannte "Tröpfeler", auf.

Anwendung der Einlage sehr einfach, indem sie im Schritt der Unterhose in der unteren Hälfte nach dem Ablösen des Schutzstreifens 11 auf einfache Weise eingeklebt werden kann. Auf diese Art und Weise hält die Einlage für einen ganzen Tag. Zudem sorgt die spezielle Form der Einlage wie bereits erwähnt dafür, dass sie unter dem Einfluss der Unterhose und der Körperwärme eine Schalenform einnimmt und der Penis somit optimal in der Mitte gehalten wird und kein austretender Urin die Unterhose oder gar die Hose beeinträchtigt. Die saugfähige Mittelschicht 10 der Einlage ist sehr weich und saugfähig. Sie vermittelt dadurch dem Benutzer den ganzen Tag ein angenehmes Gefühl von Wohlbehagen sowie von optimaler Hygiene, weil die Feuchtigkeit nach innen geleitet wird und nicht mehr auf der Haut spürbar ist. Durch das nach innen Leiten der Flüssigkeit erreicht man nämlich, dass das auf die saugfähige Mittelschicht 10 gelegte Vlies 6 trocken bleibt. Zudem schwitzt der Benutzer infolge der relativ dünnen Einlage nicht. Schliesslich wird dank der besonderen Ausgestaltung der erfindungsgemässen Einlage 1 ein Urinieren auch auf Pissoiren in öffentlichen Anlagen möglich. Diese Vorteilskombination weist keine der bereits bekannten Hilfsmittel für leicht inkontinente Männer, sogenannte "Tröpfeler", auf.

## Patentansprüche

1. Hygiene-Einlage für Männer, insbesondere für leicht inkontinente Männer, mit wenigstens einer saugfähigen Schicht (10) und einer auf dieser vorgesehenen, äusseren Schicht (8), wobei die wenigstens eine saugfähige Schicht (10) einschliesslich der äusseren Schicht (8) ellipsenartig ausgestaltet ist und auf mehreren Seiten einen v-förmigen Einschnitt (4) aufweist, **dadurch gekennzeichnet, dass** die wenigstens eine saugfähige Schicht (10) einschliesslich der äusseren Schicht (8), welche als wasserdichte Schicht oder hydrophobes Vlies ausgebildet ist, in Form einer unregelmässigen Ellipse ausgestaltet ist, dass auf den langen Seiten der Ellipse je zwei und auf der unteren kurzen Seite (3) der Ellipse ein v-förmiger Einschnitt (4) vorgesehen und die obere kurze Seite (2) der Ellipse halbmondförmig ausgespart ist, so dass die Einlage (1) unter dem Einfluss der Körperwärme und der Form der Unterhose eine schalenförmige Vertiefung bildet, und dass auf die Aussenseite der äusseren Schicht (8) wenigstens teilweise eine Klebefläche (9) aufgebracht ist.

2. Einlage nach Anspruch 1, **gekennzeichnet durch** die äussere, luftdurchlässig ausgebildete Schicht (8), die auf der Innenseite dieser Schicht (8) angebrachte mittlere, saugfähige Schicht (10) und ein die innerste Schicht bildendes Vlies (6).

3. Einlage nach Anspruch 1 oder 2, **dadurch gekennzeichnet, dass** die Klebefläche (9) streifenartig und dadurch luftdurchlässig ausgebildet ist.

4. Einlage nach Anspruch 3, **dadurch gekennzeichnet, dass** die Klebefläche (9) im ungebrauchten Zustand mittels eines abziehbaren Schutzstreifens (11), vorzugsweise aus Silikonpapier, bedeckt ist.

5. Einlage nach einem der vorhergehenden Ansprüche, **dadurch gekennzeichnet, dass** die saugfähige Schicht (10) derart ausgebildet ist, dass die auf diese Schicht (10) auftreffende Flüssigkeit nach innen geleitet wird.

6. Einlage nach einem der vorhergehenden Ansprüche, **dadurch gekennzeichnet, dass** die saugfähige Schicht (10) luftdurchlässig ausgebildet ist und superabsorbierende Fasern aufweist.

7. Einlage nach einem von Ansprüche 2 bis 6, **dadurch gekennzeichnet, dass** das die innerste Schicht bildende Vlies (6) hydrophil und die äussere Schicht (8) hydrophob ausgebildet sind.

## Claims

1. A hygiene liner for men, in particular for slightly incontinent men, comprising at least one absorptive layer (10) and an outer layer (8) provided thereon, wherein the at least one absorptive layer (10), including the outer layer (8), being in the form of an ellipse and having a v-shaped incision (4) on a plurality of sides, **characterised in that** the at least one absorptive layer (10), including the outer layer (8), which is a waterproof layer or a hydrophobic nonwoven, is in the form of an irregular ellipse, that two v-shaped incisions are provided on each of the long sides of the ellipse and one v-shaped incision on the lower short side (3) of the ellipse, and the upper short side (2) of the ellipse is cut away in a crescent-shaped manner, so that the liner forms a shell-shaped depression under the influence of body heat and the shape of the shorts, and that on the outside of the outer layer (8) at least partially an adhesive surface (9) is arranged.

2. Liner as claimed in claim 1, comprising the air-permeable outer layer (8), the middle, absorptive layer (10) applied to the inside of the said layer (8), and the nonwoven forming the innermost layer (6).

3. Liner as claimed in claim 1 or 2, **characterised in that** the adhesive surface (9) is applied, preferably in the form of strips and therefore is formed in an air-permeable manner.

4. Liner as claimed in claim 3, **characterised in that** the adhesive surface (9) in the unused state is covered by means of a peelable protective strip (11), preferably comprising silicone paper.

5. Liner as claimed in any of the preceding claims, **characterised in that** the absorptive layer (10) is formed in such a way that the fluid emerging onto said layer is guided inward.

6. Liner as claimed in any of the preceding claims, **characterised in that** the absorptive layer (10) is air-permeable and comprises superabsorbent fibers.

7. The liner as claimed in one of the claims 2 to 6, **characterised in that** the nonwoven (6), which forms the inner layer, is hydrophilic, and the outer layer (8) is hydrophobic.

## Revendications

1. Serviette hygiénique destinée aux hommes, notamment pour les hommes souffrant d'incontinence légère, munie d'au moins une couche absorbante (10) et d'une couche (8) extérieure, prévue sur celle-ci, au moins la couche absorbante (10) ainsi que la couche extérieure (8) étant en forme d'ellipse et présentant une entaille en forme de V (4) sur plusieurs côtés, **caractérisée en ce qu**'au moins une couche absorbante (10) ainsi que la couche extérieure (8), laquelle est formée comme couche imperméable aux liquides ou comme non-tissé hydrophobe, est sous forme d'ellipse irrégulière, **en ce que** respectivement deux entailles (4) en forme de V sont prévues sur les côtés longs de l'ellipse et qu'une entaille en forme de V (4) est prévue sur le côté inférieur court (3) de l'ellipse, et que le côté supérieur court (2) de l'ellipse est évidé en forme de croissant, de sorte que la serviette (1) forme, sous l'effet de la chaleur corporelle et de la forme du sous-vêtement, un creux en forme de coque, et **en ce qu**'une surface adhésive (9) est appliquée sur le côté extérieur de la couche extérieure (8), au moins partiellement.

2. Serviette selon la revendication 1, **caractérisée par** la couche extérieure (8), formée de manière perméable à l'air, la couche absorbante (10) intermédiaire, appliquée sur le côté intérieur de cette couche (8), et un non-tissé (6) formant la couche la plus à l'intérieur.

3. Serviette selon la revendication 1 ou 2, **caractérisée en ce que** la surface adhésive (9) est réalisée en forme de bande et ainsi perméable à l'air.

4. Serviette selon la revendication 3, **caractérisée en ce que** la surface adhésive (9) est recouverte, à l'état non utilisé, par une bande protectrice (11) retirable, de préférence en papier siliconé.

5. Serviette selon l'une des revendications précédentes, **caractérisée en ce que** la couche absorbante (10) est formée de telle sorte que le liquide arrivant sur cette couche (10) est dirigé vers l'intérieur.

6. Serviette selon l'une des revendications précédentes, **caractérisée en ce que** la couche absorbante (10) est formée de manière perméable à l'air et présente des fibres superabsorbantes.

7. Serviette selon l'une des revendications 2 à 6, **caractérisée en ce que** le non-tissé (6) formant la couche la plus à l'intérieur est formé de manière hydrophile et que la couche extérieure (8) est formée de manière hydrophobe.
